# EUROPEAN PATENT SPECIFICATION

(11) **EP 3 353 542 B1**
(45) Date of publication and mention of the grant of the patent: **19.06.2024**
(21) Application number: 16791438.1
(22) Date of filing: 26.09.2016
(51) Int. Cl.: G01N 33/00

(54) **MODULAR POLE SENSOR FOR VINEYARDS**
MODULARER POLSENSOR FÜR WEINBERGE
CAPTEUR MODULAIRE SOUS FORME DE PIQUET POUR VIGNOBLES

(30) Priority: 25.09.2015 PT 2015108841
(43) Date of publication of application: 01.08.2018
(73) Proprietor: Instituto Pedro Nunes - Associação Para a Inovação e Desenvolvimento em Ciência e Tecnologia, 3030-199 Coimbra (PT)
(72) Inventor: LINDO JEGUNDO DA CUNHA, António Alberto, 3030-199 Coimbra (PT); MIRANDA DIAS, Jorge Manuel, 3030-199 Coimbra (PT); ALVES PARDAL, André Filipe, 3030-199 Coimbra (PT); BAPTISTA CORREIA DA SILVA, Carlos Miguel, 3030-199 Coimbra (PT); FERREIRA SARAIVA DA MOUTA DIAS, João Paulo, 3030-199 Coimbra (PT)
(74) Representative: Patentree
(86) International application number: PCT/IB2016/055750
(87) International publication number: WO 2017/051393

(56) References cited:
- EP-A1- 2 883 445
- US-A1- 2007 144 069
- US-B1- 6 402 031
- DATABASE WPI Week 201576 Thomson Scientific, London, GB; AN 2015-65441M XP002765248, & CN 204 649 240 U (HUAIYIN TECHNOLOGY INST) 16 September 2015 (2015-09-16)
- DATABASE WPI Week 201341 Thomson Scientific, London, GB; AN 2013-H64333 XP002765249, & CN 202 735 831 U (ZHEJIANG COMML TECHNICIAN INST) 13 February 2013 (2013-02-13) cited in the application
- DATABASE WPI Week 200004 Thomson Scientific, London, GB; AN 2000-043218 XP002765250, & JP H11 304473 A (SANYO SOKKI KK) 5 November 1999 (1999-11-05)

## Description

### Technical field

The present disclosure relates to wireless sensors, in particular for sensor networks, for monitoring fruit production, in particular vineyards.

### Background Art

Document CN202735831 discloses an intelligent monitoring device aimed at monitoring environment, and temperature and humidity information of soil, in real time in an existing vineyard. The device comprises a plurality of sensors, acquiring and controlling devices, wireless communication for communicating with similar devices. Each sensor is connected with a wireless data acquisition unit which is provided with a wireless communication chip. The system is provided with the wireless communication which communicates with the wireless data acquisition units.

Document CN202735831 does not disclose arrangements for the sensor or sensor acquisition units. EP2883445 discloses a modular wireless soil measurement apparatus comprising a plurality of sensors and/or a plurality of connectors to form a sensing pole for deployment of the sensors to accommodate various underground conditions.

These facts are disclosed in order to illustrate the technical problem addressed by the present disclosure.

### General Description

The present disclosure relates to a modular ground and air pole sensor for monitoring fruit production or a vineyard comprising a plurality of sensor modules coupled to form a pole divided into a below-ground part and an above-ground part, each said sensor module comprising a tubular body; a male coupling for coupling to another of said sensor modules; and a female coupling for receiving a male coupling from another of said sensor modules. It has been found that measuring critical fruit or vine parameters, at both below and above ground, at easily configurable depths and heights, by simply adding or removing pole modules, is highly advantageous to fruit farmers and wine makers.

The pole can be divided into the below-ground and above-ground parts at the coupling between two pole sensor modules.

Alternatively, the pole can be divided into the below-ground and above-ground parts by a 'blind' pole sensor module (i.e. a sensor pole module without any sensor). This is advantageous for the robustness and water-tightness it provides at the above/below ground interface.

According to the present invention, the pole has a first sensor module, of the below-ground part, comprising a sensor for sensing ground humidity; and a plurality of second sensor modules, of the above-ground part, each of the second sensor modules of the above-ground part comprising a sensor for sensing air humidity.

It has been found that determining humidity, simultaneously below and above ground, at easily configurable depth and height, is a critical advantage for this type of fruit and vine growing.

Advantageously, said first sensor module may comprise a lateral window for the sensor sensing the ground humidity through said window.

Furthermore, an additional below-ground sensor module may be provided for sensing ground humidity at a different ground depth than the first sensor module.

Other sensors may be advantageously combined making use of the above and below ground structure of the sensor pole.

According to the present invention, a sensor module, of the above-ground part, comprises a sensor for sensing leaf humidity, a sensor module, of the above-ground part, further comprises a sensor for sensing rainfall precipitation.

In an embodiment, a sensor module, of the above-ground part, comprises a sensor for sensing air temperature.

In accordance with the present invention, a sensor module, of the above-ground part, comprises a sensor for sensing air pressure; a sensor module, of the above-ground part, further comprises a sensor for sensing wind speed or for sensing wind speed and direction; sensor module, of the above-ground part, further comprises a sensor for sensing visible-light solar radiation and UV solar radiation.

In an embodiment, the tubular bodies of the sensor modules may have the same length.

In an embodiment, the male coupling may be bonded to the tubular body, in particular the male coupling is glued to the tubular body.

In an embodiment, one or more of the sensor modules may comprise:
a first electric connector at the male coupling for connecting with another sensor module;
a second electric connector at the female coupling for connecting with another sensor module; and
a set of electric conductors connecting said first and said second connectors.

In an embodiment, the first electric connector, the second electric connector and the set of electric conductors may comprise a ground connection, a supply voltage connection and at least one data connection.

In an embodiment, the male and female couplings may be tubular.

In an embodiment, the male or the female coupling may comprise one or more o-rings and one or more grooves for receiving said o-rings for sealing the male-female coupling, in particular the grooves may be transversal to the tubular coupling.

In an embodiment, the male or female coupling may comprise a groove for receiving an electric cable for connecting between inside and outside of the sensor module, in particular the grooves may be longitudinal to the tubular coupling.

In an embodiment, the male and female couplings may be tubular and the male coupling may be a tube with a smaller diameter.

In an embodiment, one or more of the sensor modules may comprise a cylindrical ring coupled to the male coupling with an opening for passage of an electrical cable.

In an embodiment, one or more of the sensor modules may comprise a protruding support for holding a sensor or photovoltaic panel.

In an embodiment, the protruding support may comprise a support rail, in particular for receiving sensor PCBs.

In an embodiment, the protruding support, previously described, may be attached to and protruding from the cylindrical ring.

In an embodiment, the tubular body of the sensor module and the tubular male and female couplings may have a round or square cross-section.

In an embodiment, the sensor module of the modular pole sensor may comprise two or more layers of padding inside the sensor module for receiving an electronic circuit between two of said layers, in particular for affixing the electronic circuit between said layers, further in particular the padding being polyurethane foam or expanded polypropylene.

In an embodiment, the modular pole sensor may further comprise a terminal sensor module having a conical termination for insertion into the ground.

An embodiment comprises wireless communications.

In an embodiment, the modular pole sensor may further comprise a terminal sensor module having a wireless communication antenna and a cap for capping the tubular body, in particular the cap may be bonded to the tubular body.

In an embodiment, the modular pole sensor may have a sensor and a lateral window for sensing ground humidity.

Throughout the description and claims the word "comprise" and variations of the word, are not intended to exclude other technical features, additives, components, or steps. Additional objectives, advantages and features of the solution will become apparent to those skilled in the art upon examination of the description or may be learned by practice of the solution.

### Brief Description of the Drawings

The following figures provide preferred embodiments for the present disclosure and should not be seen as limiting the scope of the disclosure.
**Figure 1****:** Simple tubular module machined in order to allow the modularity of the present disclosure, wherein 1 represents surface; 2 represents a female connection interface; 3 represents a male connection interface; 4 represents a carving to the interface cable; 5 represents a carving for the o-ring of tightness.
**Figure 2****:** Male/male connection that also support the electronic components, wherein 6 represents a groove for the interface cable; 7 represents a groove for the o-rings of fluid-tightness.
**Figure 3****:** Male/male connection with installed pipe section, wherein 8 represents a section of connection pipe between modules; 9 represents a male connections for interface with the pipes.
**Figure 4****:** Interface ring between the inside and outside of the modules, wherein 10 represents stretch marks to facilitate the installation of the ring in connections; 11 represents an opening to allow performing the passage of a cable.
**Figure 5****:** Interface ring between the inside and the outside of the modules with the holder for the sensors and solar panel, wherein 12 represents a holder for fixation of the sensors and the solar panel; 13 represents a flat surface for fixation of the sensors; 14 represents cavities for fixing screws; 15 represents a fixation surface of the sensors at 45°; 16 represents as rail for sensors installed on PCB.
**Figure 6****:** Support structure to the PCB "sandwich" type in EPP (expanded polypropylene) composed by two halves, wherein 17 represents cavities for the electronic components; 18 represents a carving corresponding to the module of male/male connection; 19 represents EPP halves to involve the electronic components.
**Figure 7****:** Holder in EPP for the ground sensors with conical ending, wherein 20 represents a male connection to link to pipe; 21 represents a ground sensor installed; 22 represents a conical ending.
**Figure 8****:** Holder in EPP for the ground sensors with interface for pipe at both ends, wherein 23 represents an upper male connection to link to pipe; 24 represents a lower male connection to link to pipe.

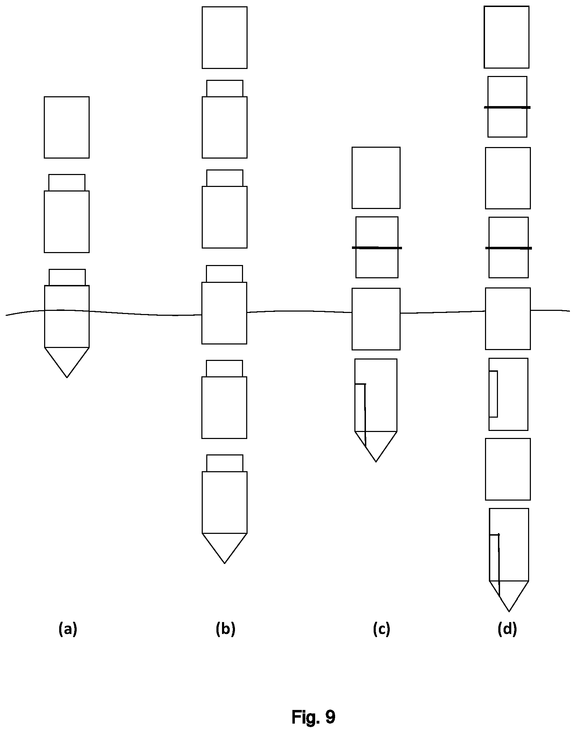

**Figure 9****:** Several representations of possible embodiments of the disclosure.

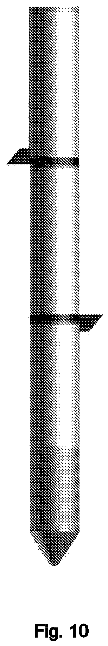

**Figure 10****:** Representation of an embodiment of the disclosure.

### Detailed Description

In an embodiment, the present disclosure may comprise underground modularity, meaning that the possibility of modularity is not only above the ground but as well as below.

In an embodiment, this disclosure comprises the possibility of measuring several sensory parameters at variable depths according to the application, in other words, according to the necessity it is possible to change the monitoring depth just by changing the dimension of the support pipe.

In an embodiment, the disclosure may either be installed with no sensor, with one sensor, or more than one sensor, depending of the intended application.

In an embodiment wherein no sensor is installed the bottom of the device, the conic ending, is used as a holder.

In an embodiment, the installation of the solar panels may be performed laterally or on a support. The disclosure is completely autonomous regarding the energy level, for that it makes use of solar panels that recharge the batteries which power the entire system.

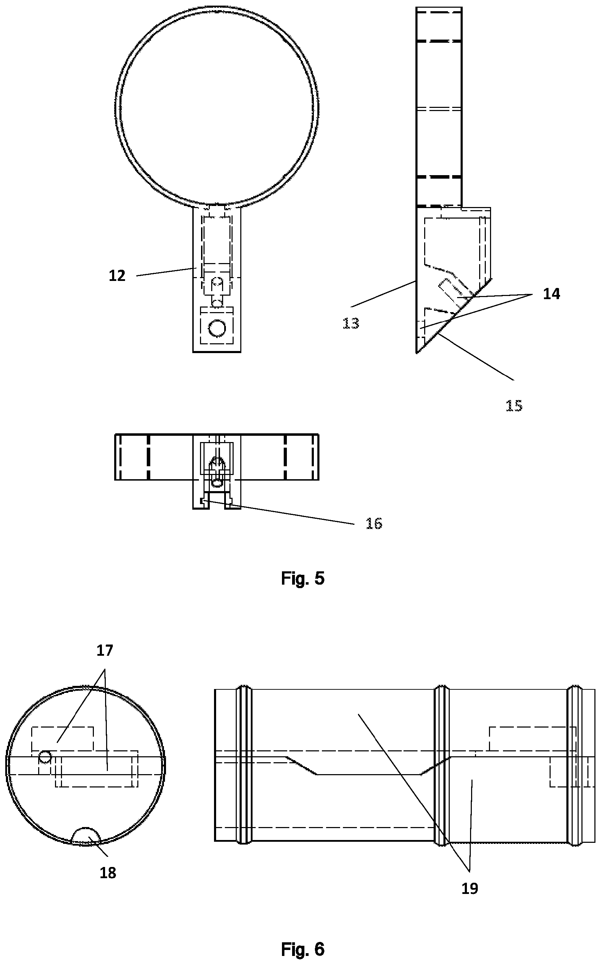

In an embodiment, the possibility of installing solar panels in the devices may be performed in two distinct forms, namely, through fixing in holder designed for the purpose, represented in Figure 5, or by fixing it directly in the tubular body of the encapsulation using for that flexible solar panels.

The construction variables may be described as follows. For the assembly of the device the following possibilities are considered according to the application and respective product.

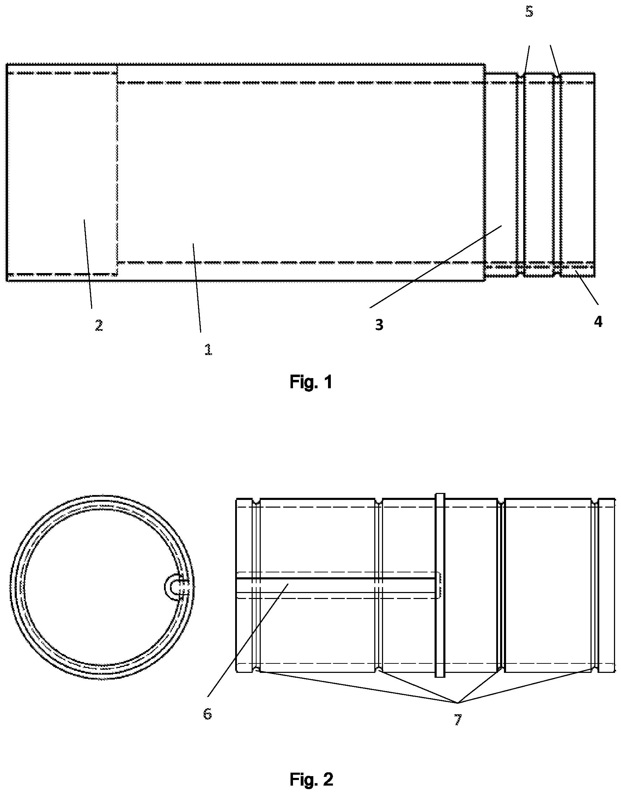

In an embodiment, the modular structure developed from a sectioned common pipe and which is machined forming male and female connections that interlock (Figure 1), presenting a conical termination on the lower module and a lid as termination of the upper module. It is further referred, the three carvings that the male union contemplates, where the two transversal allow installing two o-rings which ensure tightness and fixation, while the third longitudinal allows the passage of the interface cable with the outside without compromising that same tightness of the system.

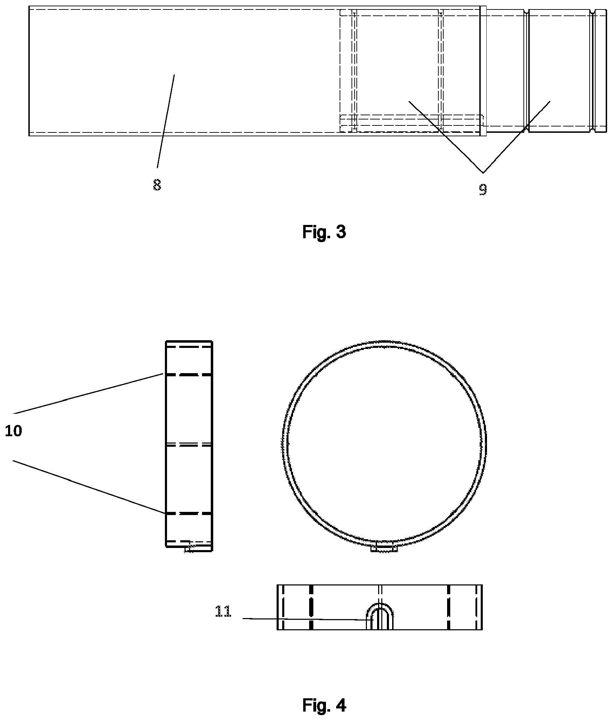

In an embodiment, the male/male connection which also serves as holder for the electronics, as well as can support the interface ring between the interior and exterior of each of the modules (Figure 2). In this structure the pipe is used in its simple form (without any kind of machining, being just cut perpendicularly according to the application) and allows defining the distance in height between modules/connections (Figure 3). Thus, it is possible to effect the customization of the system according to the needs of each application. It is further noted that this structure comprises five carvings, being four transverse and allow the installation of four o-rings respectively, ensuring the tightness and fixation, while the fifth carving is longitudinal and allows the passage of the interface cable with the outside without compromising the tightness of the system.

In an embodiment, the interface ring has two possible shapes, a simpler which serves only to allow performing the interface with the exterior of cables for connection of sensors or actuators (Figure 4), and other more complex that, in addition to the cables interface, also allows to perform the support of several sensors and of the solar panel (Figure 5). Also note that these structures can either be installed on the male/male connection (Figure 2), as well as on the male interface structure machined (Figure 1).

In an embodiment, the support structure and filling of the modules of EPP, composed by two halves which involve the PCBs of the several modules in the form of "sandwich" type (Figure 6). After the association of this structure to the PCBs, the same is introduced under pressure in the tubular modules whether in male/male connection (Figure 2) or in the machined structure (Figure 1).

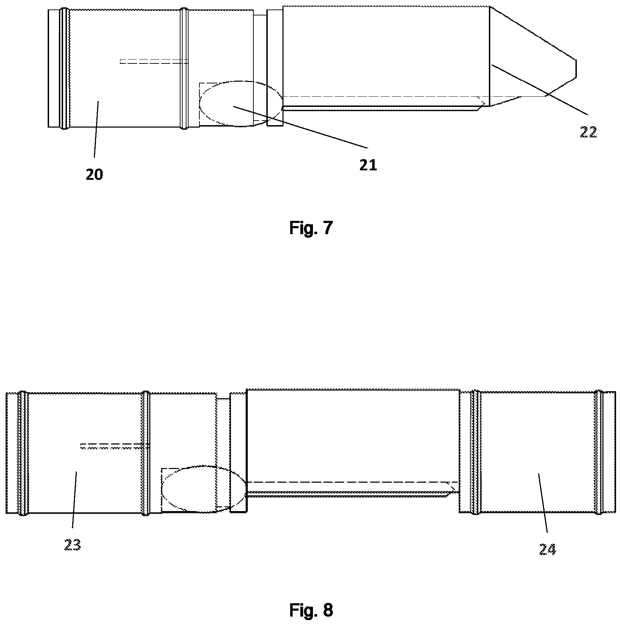

In an embodiment, the ground monitoring sensors still have a supporting structure in EPP, also composed by two halves that comprise two possible configurations, namely the support structure and conical bottom termination (Figure 7) and the support and connection structure to the next sensor (Figure 8), with interface for pipe on both ends. Thus, it is possible to make also at the ground level the customization of the system according to the needs.

The present disclosure should not be seen in any way restricted to the embodiments described and a person with ordinary skill in the art will foresee many possibilities to modifications thereof.

The above described embodiments are combinable. The following claims further set out particular embodiments of the disclosure.

## Claims

1. Modular ground and air pole sensor for monitoring fruit production or a vineyard comprising:
a plurality of sensor modules coupled to form a pole divided into a below-ground part comprising a first sensor module and an above-ground part comprising a plurality of second sensor modules, each said sensor module comprising:
a tubular body;
a male coupling for coupling to another of said sensor modules;
a female coupling for receiving a male coupling from another of said sensor modules,
the first sensor module, of the below-ground part, comprises a ground humidity sensor; and
**characterised in that**
each of the second sensor modules, of the above-ground part, comprises an air humidity sensor, a sensor for sensing air pressure, a sensor for sensing wind speed or for sensing wind speed and direction, a sensor for sensing visible-light solar radiation and UV solar radiation, a sensor for sensing leaf humidity, a sensor for sensing rainfall precipitation, or combinations thereof.

2. Modular pole sensor according to any previous claims wherein each of a plurality of the sensor modules comprises: a first electric connector at the male coupling for connecting with another sensor module; a second electric connector at the female coupling for connecting with another sensor module; a set of electric conductors connecting said first and said second connectors.

3. Modular pole sensor according to any previous claim wherein the first sensor module comprises a lateral window for the sensor sensing ground humidity through said window.

4. Modular pole sensor according to any previous claim wherein the male and female couplings are tubular.

5. Modular pole sensor according to the previous claim wherein the male or the female coupling comprises one or more o-rings and one or more grooves for receiving said o-rings for sealing the male-female coupling, in particular the grooves being transversal to the tubular coupling.

6. Modular pole sensor according to any previous claim wherein the male or female coupling comprises a groove for receiving an electric cable for connecting between inside and outside of the sensor module, in particular the grooves being longitudinal to the tubular coupling.

7. Modular pole sensor according to any previous claim wherein the male and female couplings are tubular, the male coupling is a tube with a smaller diameter.

8. Modular pole sensor according to any previous claim wherein one or more of the sensor modules comprise a cylindrical ring coupled to the male coupling with an opening for passage of an electrical cable, wherein a sensor module comprises a sensor connected to an electrical cable and said ring is for supporting the sensor and said opening for passage of the electrical cable is for connecting electrical cable to the electric connectors, if present.

9. Modular pole sensor according to any previous claim wherein one or more of the sensor modules comprise a protruding support for holding a sensor or photovoltaic panel.

10. Modular pole sensor according to the previous claim wherein the protruding support comprises a support rail for receiving sensor PCBs.

11. Modular pole sensor according to claims 8 and 10, wherein the protruding support is attached to and protruding from the cylindrical ring.

12. Modular pole sensor according to any of the previous claims wherein the tubular body of the sensor module and the tubular male and female couplings have a round or square cross-section.

13. Modular pole sensor according to any previous claim wherein the sensor module comprises two or more layers of padding inside the sensor module for receiving an electronic circuit between two of said layers, in particular for affixing the electronic circuit between said layers, further in particular the padding being polyurethane foam or expanded polypropylene.

14. Modular pole sensor according to any previous claim comprising a terminal sensor module having a conical termination for insertion into the ground.

15. Modular pole sensor according to any previous claim comprising a terminal sensor module having a wireless communication antenna and a cap for capping the tubular body, in particular the cap being bonded to a tubular body.

## Patentansprüche

1. Modularer Boden- und Luftstangensensor zur Überwachung der Obstproduktion oder eines Weinbergs, umfassend:
eine Vielzahl von Sensormodulen, die so gekoppelt sind, dass sie eine Stange bilden, die in einen unterirdischen Abschnitt mit einem ersten Sensormodul und einen oberirdischen Abschnitt mit einer Vielzahl von zweiten Sensormodulen unterteilt ist, wobei jedes genannte Sensormodul umfasst:
einen röhrenförmigen Körper;
eine Steckerkupplung zur Verbindung mit einem anderen der genannten Sensormodule;
eine Steckkupplung zur Aufnahme einer Steckerkupplung der anderen genannten Sensormodule,
das erste Sensormodul des unterirdischen Abschnitts, umfassend einen Bodenfeuchtesensor; und
**dadurch gekennzeichnet, dass** jedes der zweiten Sensormodule des oberirdischen Abschnitts einen Luftfeuchtigkeitssensor, einen Sensor zur Erfassung des Luftdrucks, einen Sensor zur Erfassung der Windgeschwindigkeit oder zur Erfassung der Windgeschwindigkeit und Windrichtung, einen Sensor zur Erfassung der sichtbaren Sonnenstrahlung und der UV-Sonnenstrahlung, einen Sensor zur Erfassung der Blattfeuchte, einen Sensor zur Erfassung des Niederschlags oder Kombinationen davon umfasst.

2. Modularer Stangensensor nach einem der vorangehenden Ansprüche, wobei jede einer Vielzahl der Sensormodule umfasst: einen ersten elektrischen Anschluss an der Steckerkupplung für den Anschluss an ein anderes Sensormodul; einen zweiten elektrischen Anschluss an der Steckkupplung für den Anschluss an ein anderes Sensormodul; einen Satz elektrischer Leiter, die den genannte ersten und den genannten zweiten Anschluss verbinden.

3. Modularer Stangensensor nach einem der vorangehenden Ansprüche, wobei das erste Sensormodul eine seitliche Öffnung für den Sensor umfasst, der die Bodenfeuchtigkeit durch die genannte Öffnung erfasst.

4. Modularer Stangensensor nach einem der vorangehenden Ansprüche, wobei die Steckerkupplung und die Steckkupplung röhrenförmig sind.

5. Modularer Stangensensor nach dem vorangehenden Anspruch, wobei die Steckerkupplung oder die Steckkupplung einen oder mehrere O-Ringe und eine oder mehrere Nuten zur Aufnahme der genannten O-Ringe zur Abdichtung der Stecker-SteccKupplung umfasst, wobei die Nuten insbesondere quer zu der rohrförmigen Kupplung verlaufen.

6. Modularer Stangensensor nach einem der vorangehenden Ansprüche, wobei die Steckerkupplung und die Steckkupplung eine Nut zur Aufnahme eines elektrischen Kabels zur Verbindung zwischen der Innenseite und der Außenseite des Sensormoduls umfassen, wobei die Nuten insbesondere in Längsrichtung der rohrförmigen Kupplung verlaufen.

7. Modularer Stangensensor nach einem der vorangehenden Ansprüche, wobei die Steckerkupplung und die Steckkupplung rohrförmig sind und die Steckerkupplung ein Rohr mit einem kleineren Durchmesser ist.

8. Modularer Stangensensor nach einem der vorangehenden Ansprüche, wobei eines oder mehrere der Sensormodule einen zylindrischen Ring umfassen, der mit der Steckerkupplung mit einer Öffnung für den Durchgang eines elektrischen Kabels gekoppelt ist, wobei ein Sensormodul einen Sensor umfasst, der mit einem elektrischen Kabel verbunden ist, und der genannte Ring dazu dient, den Sensor zu tragen, und die genannte Öffnung für den Durchgang des elektrischen Kabels dazu dient, das elektrische Kabel mit den elektrischen Anschlüssen zu verbinden, falls vorhanden.

9. Modularer Stangensensor nach einem der vorangehenden Ansprüche, wobei eines oder mehrere der Sensormodule eine vorstehende Halterung als Halterung für einen Sensor oder ein photovoltaisches Paneel umfassen.

10. Modularer Stangensensor nach dem vorangehenden Anspruch, wobei die vorstehende Halterung eine Trägerschiene zur Aufnahme von Sensorplatinen umfasst.

11. Modularer Stangensensor nach den Ansprüchen 8 und 10, wobei die vorstehende Halterung an dem zylindrischen Ring befestigt ist und von dem zylindrischen Ring absteht.

12. Modularer Stangensensor nach einem der vorangehenden Ansprüche, wobei der röhrenförmige Körper des Sensormoduls und die röhrenförmige Steckerkupplung und die röhrenförmige Steckkupplung einen runden oder quadratischen Querschnitt aufweisen.

13. Modularer Stangensensor nach einem der vorangehenden Ansprüche, wobei das Sensormodul zwei oder mehr Schichten eines Füllmaterials im Inneren des Sensormoduls umfasst, um eine elektronische Schaltung zwischen zwei der genannten Schichten aufzunehmen, insbesondere um die elektronische Schaltung zwischen den genannten Schichten zu befestigen, wobei das Füllmaterial ferner insbesondere aus Polyurethanschaum oder expandiertem Polypropylen besteht.

14. Modularer Stangensensor nach einem der vorangehenden Ansprüche, umfassend ein Endsensormodul mit einem konischen Abschluss zum Einführen in den Boden.

15. Modularer Stangensensor nach einem der vorangehenden Ansprüche, umfassend ein Endsensormodul mit einer Antenne für drahtlose Kommunikation und eine Kappe zum Verschließen des röhrenförmigen Körpers, wobei die Kappe insbesondere mit einem röhrenförmigen Körper verbunden ist.

## Revendications

1. Capteur modulaire de sol et d'air sous forme de piquet pour la supervision de la production de fruits ou d'un vignoble comprenant :
une pluralité de modules capteurs accouplés pour former un piquet divisé entre une partie souterraine comprenant un premier module capteur et une partie en surface comprenant une pluralité de seconds modules capteurs, chacun desdits modules capteurs comprenant :
un corps tubulaire ;
un élément de couplage mâle pour accoupler à un autre desdits modules capteurs ;
un élément de couplage femelle pour recevoir un élément de couplage mâle d'un autre desdits modules capteurs,
le premier module capteur, de la partie souterraine, comprend un capteur d'humidité de sol ; et
**caractérisé en ce que** chacun des seconds modules capteurs, de la partie en surface, comprend un capteur d'humidité de l'air, un capteur pour capter la pression de l'air, un capteur pour capter la vitesse du vent ou pour capter la vitesse et la direction du vent, un capteur pour capter le rayonnement solaire de la lumière visible et le rayonnement solaire UV, un capteur pour capter l'humidité des feuilles, un capteur pour capter la précipitation des pluies, ou des combinaisons de ceux-ci.

2. Capteur modulaire sous forme de piquet selon l'une quelconque des revendications précédentes dans lequel chacun d'une pluralité de modules capteurs comprend : un premier connecteur électrique à élément de couplage mâle pour se connecter à un autre module capteur ; un second connecteur électrique à élément de couplage femelle pour se connecter à un autre module capteur ; un ensemble de conducteurs électriques connectant ledit premier connecteur et ledit second connecteur.

3. Capteur modulaire sous forme de piquet selon l'une quelconque des revendications précédentes dans lequel le premier module capteur comprend une fenêtre latérale pour que le capteur puisse capter l'humidité du sol à travers ladite fenêtre.

4. Capteur modulaire sous forme de piquet selon l'une quelconque des revendications précédentes dans lequel les éléments de couplage mâle et femelle sont tubulaires.

5. Capteur modulaire sous forme de piquet selon la revendication précédente dans lequel l'élément de couplage mâle ou femelle comprend un ou plusieurs joints toriques et une ou plusieurs rainures pour recevoir lesdits joints toriques pour sceller l'élément de couplage mâle-femelle, les rainures étant en particulier transversales au couplage tubulaire.

6. Capteur modulaire sous forme de piquet selon l'une quelconque des revendications précédentes dans lequel l'élément de couplage mâle ou femelle comprend une rainure pour recevoir un câble électrique pour le raccordement entre l'intérieur et l'extérieur du module capteur, les rainures étant en particulier longitudinales à l'élément de couplage tubulaire.

7. Capteur modulaire sous forme de piquet selon l'une quelconque des revendications précédentes dans lequel les éléments de couplage mâle et femelle sont tubulaires, l'élément de couplage mâle étant un tube de diamètre inférieur.

8. Capteur modulaire sous forme de piquet selon l'une quelconque des revendications précédentes dans lequel un ou plusieurs des modules capteurs comprend un anneau cylindrique accouplé à l'élément de couplage mâle avec une ouverture pour le passage d'un câble électrique, dans lequel un module capteur comprend un capteur connecté à un câble électrique et ledit anneau sert à soutenir le capteur et ladite ouverture pour le passage du câble électrique sert à connecter le câble électrique aux connecteurs électriques, si présents.

9. Capteur modulaire sous forme de piquet selon l'une quelconque des revendications précédentes dans lequel un ou plusieurs des modules capteurs comprend un support saillant pour tenir un capteur ou un panneau photovoltaïque.

10. Capteur modulaire sous forme de piquet selon la revendication précédente dans lequel le support saillant comprend un rail de support pour recevoir la carte de circuit imprimée capteuse.

11. Capteur modulaire sous forme de piquet selon les revendications 8 et 10, dans lequel le support saillant est attaché à l'anneau et le dépasse.

12. Capteur modulaire sous forme de piquet selon l'une quelconque des revendications précédentes dans lequel le corps tubulaire du module capteur et les éléments de couplage tubulaires mâle et femelle ont une section transversale ronde ou carrée.

13. Capteur modulaire sous forme de piquet selon l'une quelconque des revendications précédentes dans lequel le module capteur comprend deux ou plus de couches de rembourrage à l'intérieur du module capteur pour recevoir un circuit électronique entre deux desdites couches, en particulier pour apposer le circuit électronique entre lesdites couches, plus particulièrement le rembourrage étant de la mousse de polyuréthane ou du polypropylène expansé.

14. Capteur modulaire sous forme de piquet selon l'une quelconque des revendications précédentes comprenant un module capteur terminal ayant une terminaison conique destinée à être insérée dans le sol.

15. Capteur modulaire sous forme de piquet selon l'une quelconque des revendications précédentes comprenant un module capteur terminal ayant une antenne de communication sans fil et un bouchon pour boucher le corps tubulaire, le bouchon étant en particulier lié à un corps tubulaire.
